# EUROPÄISCHE PATENTSCHRIFT

(11) **EP 0 713 854 B1**
(45) Veröffentlichungstag und Bekanntmachung des Hinweises auf die Patenterteilung: **20.05.1998**
(21) Anmeldenummer: 95117892.0
(22) Anmeldetag: 14.11.1995
(51) Int. Cl.: C07C 57/04, C07C 51/50, C07C 51/487

(54) **Verfahren zur Reinigung von Roh-(Meth)acrylsäure**
Process for purifying crude (meth)acrylic acid
Procédé de purification de l'acide (méth)acrylique brut

(30) Priorität: 23.11.1994 US 347131
(43) Veröffentlichungstag der Anmeldung: 29.05.1996
(73) Patentinhaber: BASF AKTIENGESELLSCHAFT, 67056 Ludwigshafen (DE)
(72) Erfinder: Herbst, Holger, Dr., D-67227 Frankenthal (DE); Nestler, Gerhard, Dr., D-67061 Ludwigshafen (DE); Darlington, Jerry, Lake Jackson, Texas 77566 (US); Martan, Hans, Dr., D-67227 Frankenthal (DE)

(56) Entgegenhaltungen:
- EP-A- 0 434 080
- EP-A- 0 613 924

## Beschreibung

Die vorliegende Erfindung betrifft ein Verfahren zum Abtrennen von Aldehyden aus Roh-(Meth)acrylsäure, die nach dem Verfahren der katalytischen Gasphasenoxidation erzeugt wurde, bei dem man die Roh-(Meth)acrylsäure mit einem Hydrazinderivat versetzt und aus dem Gemisch die (Meth)acrylsäure destillativ abtrennt.

(Meth)acrylsäure wird als verkürzte Schreibweise verwendet und steht für Acrylsäure oder Methacrylsäure.

(Meth)acrylsäure, entweder für sich oder in Form ihrer Ester, ist insbesondere zur Herstellung von Polymerisaten für die verschiedensten Anwendungsgebiete, z.B. Verwendung als Klebstoffe, von Bedeutung.

Unter anderem ist (Meth)acrylsäure durch katalytische Gasphasenoxidation von Alkanen, Alkanolen, Alkenen oder Alkenalen erhältlich, die 3 bzw. 4 C-Atome enthalten. Besonders vorteilhaft ist (Meth)acrylsäure z.B. durch katalytische Gasphasenoxidation von Propen, Acrolein, tert.-Butanol, iso-Buten, iso-Butan, iso-Butyraldehyd oder Methacrolein erhältlich. Als Ausgangsverbindungen sind aber auch solche denkbar, aus welchen sich die eigentliche C₄-Ausgangsverbindung während der Gasphasenoxidation erst intermediär bildet. Beispielhaft genannt sei der Methylether des tert.-Butanols. Dabei werden diese Ausgangsgase, in der Regel mit inerten Gasen wie Stickstoff, CO₂, gesättigten Kohlenwasserstoffen und/oder Wasserdampf verdünnt, im Gemisch mit Sauerstoff bei erhöhten Temperaturen sowie gegebenenfalls erhöhtem Druck über übergangsmetallische Mischoxidkatalysatoren geleitet, oxidativ in die (Meth)acrylsäure umgewandelt und durch die Aufnahme in ein geeignetes Absorptionsmittel (z.B. Wasser oder ein Gemisch aus 70 bis 75 Gew.-% Diphenylether und 25 bis 30 Gew.-% Diphenyl) aus dem Produktgasstrom abgetrennt (vgl. z.B. EP-A 297 445 und DE-PS 21 36 396).

Nach Entfernung des Absorptionsmittels (und gegebenenfalls zuvor erfolgter Desorption von eine geringe Absorptionsmittellöslichkeit aufweisenden Verunreinigungen durch Abstreifen, z.B. mit Luft) über extraktive und/oder destillative Trennverfahren (z.B. Entfernung des Absorptionsmittels Wasser durch Destillation, azeotrope Destillation oder extraktive Abtrennung der Säure aus der wäßrigen Lösung und anschließende destillative Entfernung des Extraktionsmittels) wird eine Säure erhalten, die hier als Roh-(Meth)acrylsäure bezeichnet wird.

Aufgrund zahlreicher im Verlauf der katalytischen Gasphasenoxidation erfolgenden Parallel- und Folgereaktionen bildet die Roh-(Meth)acrylsäure kein reines Produkt. Vielmehr enthält sie ein Spektrum verschiedener Verunreinigungen (in der Regel in der Größenordnung von ≤ 2 Gew.-%, vgl. EP-B 169 254), das hauptsächlich aus mit den Ausgangsverbindungen der katalytischen Gasphasenoxidation und mit der resultierenden (Meth)acrylsäure chemisch verwandten Aldehyden besteht. In der Regel enthält Roh-(Meth)acrylsäure daher als Verunreinigungen neben Essig-, Ameisen- und Propionsäure in der Regel Formaldehyd, Acetaldehyd, Acrolein, Methacrolein, Propionaldehyd, n-Butyraldehyd, Benzaldehyd, Furfural und Crotonaldehyd.

Für verschiedene Anwendungen der (Meth)acrylsäure wirken sich die in der Roh-(Meth)acrylsäure enthaltenen Verunreinigungen nachteilig aus (vgl. z.B. DE-AS 22 07 184). So kann z.B. die Induktionszeit von Polymerisationsrektionen, d.h. der Zeitraum zwischen dem Erreichen der Polymerisationstemperatur und dem tatsächlichen Beginn der Polymerisation, nicht reproduzierbar oder der Polymerisationsgrad reduziert sein. Auch können die Polymerisate zum Verfärben neigen.

Deshalb ist man für solche Anwendungszwecke bestrebt, die Verunreinigungen aus der Roh-(Meth)acrylsäure weitgehend abzutrennen und Roh-(Meth)acrylsäure in Rein-(Meth)acrylsäure überzuführen. Dies erfolgt in der Regel auf destillativem Weg, z.B. durch zwei sich anschließende Rektifikationsstufen zur Entfernung leichter und schwerer als die (Meth)acrylsäure siedender Verunreinigungen (vgl. z.B. EP-B 102 642).

Problematisch ist jedoch, daß wenigstens ein Teil der aldehydischen Verunreinigungen in seinem physikalischen Verhalten der (Meth)acrylsäure so ähnlich ist, daß eine ausschließlich rektifikative Entfernung derselben nur unter Anwendung einer unwirtschaftlichen Anzahl von Trennböden und/oder eines unwirtschaftlichen Rücklaufverhältnisses möglich ist.

Aus der GB-PS 1 346 737 und aus der DE-B 22 07 184 ist daher ein Verfahren zur Reinigung von Roh-Acrylsäure bekannt, das dadurch gekennzeichnet ist, daß man der Roh-Acrylsäure Verbindungen zusetzt, die eine -NH₂-Gruppe aufweisen und aus dem Gemisch die Acrylsäure destillativ abtrennt. Die primären Amine binden die als Verunreinigungen enthaltenen Aldehyde in hohem Ausmaß, so daß bereits eine sich anschließende einfach destillative Trennstufe bezüglich der aldehydischen Verunreingungen eine hohe Trennwirkung erzielt. Die kleinsten Restgehalte an Aldehyden werden dabei mit Hydrazin und Phenylhydrazin als aminischen Verbindungen erzielt. Nachteilig an einer Anwendung der letztgenannten Verbindungen ist jedoch, daß Hydrazin und Phenylhydrazin toxikologisch nicht unbedenklich sind. Die EP-A 270 999 empfiehlt daher Roh-(Meth)acrylsäure vor der destillativen Aufarbeitung mit Guanylhydrazin (Aminoguanidin) und/oder dessen Salzen (vorzugsweise Aminoguanidinhydrogencarbonat) in Mengen von 1 bis 3 Mol pro Mol enthaltenem Aldehyd zu versetzen. Nachteilig an Aminoguanidin und/oder dessen Salzen ist jedoch, daß die Geschwindigkeit ihrer Umsetzung mit Aldehyden nicht voll zu befriedigen vermag. Die DE-A 43 35 172 empfiehlt, vor der destillativen Aufarbeitung zusätzlich eine organische Sulfonsäure zuzusetzen, um Belagsbildung zu vermeiden. Die JP-A 117 716/75 empfiehlt bei Abwesenheit von Aminverbindungen die Kombination Phenothiazin/Sulfonsäure, um die Polymerisationsneigung von Acrylsäure zu mindern.

Aufgabe der vorliegenden Erfindung war daher ein Verfahren zum Abtrennen von Aldehyden aus Roh-(Meth)acrylsäure, die nach dem Verfahren der katalytischen Gasphasenoxidation erzeugt wurde, bei dem man die Roh-(Meth)acrylsäure mit einem Hydrazinderivat versetzt, das die Nachteile der vorgenannten Hydrazinverbindungen des Standes der Technik nicht aufweist, und aus dem Gemisch die (Meth)acrylsäure destillativ abtrennt.

Demgemäß wurde ein Verfahren zum Abtrennen von Aldehyden aus Roh-(Meth)acrylsäure, die nach dem Verfahren der katalytischen Gasphasenoxidation erzeugt wurde, bei dem man die Roh-(Meth)acrylsäure mit einem Hydrazinderivat versetzt und aus dem Gemisch die (Meth)acrylsäure destillativ abtrennt, gefunden, das dadurch gekennzeichnet ist, daß man als Hydrazinderivat das Hydrazid einer organischen Carbonsäure verwendet.

Als solche Hydrazide organischer Carbonsäuren kommen insbesondere in Betracht: Semicarbazid (Carbamidsäurehydrazid) sowie die Mono- und Dihydrazide von 1 bis 10 C-Atome aufweisenden gesättigten aliphatischen Mono- und/oder Dicarbonsäuren. Das sind insbesondere die Hydrazide von Ameisensäure, Essigsäure, Propionsäure, Butansäure und Pentansäure. Als gesättigte aliphatische Dicarbonsäuren kommen für die entsprechenden Hydrazide insbesondere diejenigen in Betracht, die 4 bis 8 C-Atome aufweisen. Ganz besonders geeignet sind das Dihydrazid der Adipinsäure und der Bernsteinsäure. Selbstverständlich können anstelle der Carbonsäurehydrazide auch deren Salze eingesetzt werden. Als solche kommen beispielsweise deren Hydrogencarbonat, Nitrat, Sulfat oder Chlorid in Betracht, wie z.B. Semicarbazidhydrochlorid.

Die zur Bestimmung der zuzusetzenden Absolutmenge des Carbonsäurehydrazids erforderliche Kenntnis des Gehalts an Aldehyden läßt sich in dem Fachmann bekannter Weise, nach geeigneter Derivatisierung der Aldehyde, durch die Methode der Hochdruckflüssigchromathographie (HPLC) ermitteln. Pro Mol aldehydischer Verunreinigungen wird man in der Regel wenigstens 0,5 Mol, normalerweise aber nicht mehr als 5 Mol, an Carbonsäurehydrazidverbindung zusetzen. Vorzugsweise beträgt die einzusetzende Menge an Carbonsäurehydrazid, in gleicher Weise bezogen, 1 bis 3 Mol und ganz besonders bevorzugt 1 bis 2 Mol.

Bevorzugt erfolgt der Zusatz des erfindungsgemäßen Carbonsäurehydrazids erst kurz vor der destillativen Aufarbeitung. D.h. man setzt der Roh-(Meth)acrylsäure das erfindungsgemäße Carbonsäurehydrazid zu, überläßt das Gemisch einige Zeit sich selbst (die Reaktionsdauer hängt von der Temperatur ab; bei Temperaturen von 20 bis 100°C liegt sie zwischen wenigen Minuten und einigen Stunden, anwendungstechnisch vorteilhaft erfolgt das Sichselbstüberlassen bei Raumtemperatur) und arbeitet anschließend destillativ auf. Die erfindungsgemäßen Carbonsäurehydrazide können sowohl in Substanz als auch in Lösung (als Lösungsmittel eignen sich z.B. Wasser oder (Meth)acrylsäure) zugesetzt werden.

Eine gewisse Beeinträchtigung des erfindungsgemäßen Verfahrens tritt häufig insofern auf, daß sich während der destillativen Abtrennung die Destillationsvorrichtungen (insbesondere die Verdampferoberfläche) relativ rasch mit Belag bedecken, der durch das Beisein der erfindungsgemäßen Carbonsäurehydrazide und/oder deren Salze bedingt wird, da er bei einer destillativen Aufarbeitung der Roh-(Meth)acrylsäure in Abwesenheit dieser Zusätze nicht auftritt (allerdings bedingt eine solche zusatzfreie destillative Aufarbeitung nur eine geringe Trennwirkung bezüglich der in der Roh- (Meth) acrylsäure enthaltenen aldehydischen Verunreinigungen).

Offensichtlich sind an der Belagsbildung unmittelbare Umsetzungsprodukte der Carbonsäurehydrazide und/oder deren Salze mit den aldehydischen Verunreinigungen und/oder im Rahmen der destillativen Aufarbeitung aus diesen entstehende Folgeprodukte beteiligt.

Aufgabe der vorliegenden Erfindung war daher auch ein Verfahren zum Abtrennen von Aldehyden aus Roh-(Meth)acrylsäure, die nach dem Verfahren der katalytischen Gasphasenoxidation erzeugt wurde, bei dem die beschriebene Belagsbildung gemindert wird.

Demgemäß wurde auch ein Verfahren zum Abtrennen von Aldehyden aus Roh-(Meth)acrylsäure, die nach dem Verfahren der katalytischen Gasphasenoxidation erzeugt wurde, bei dem man die Roh-(Meth)acrylsäure mit erfindungsgemäßem Carbonsäurehydrazid und/oder dessen Salzen versetzt und aus dem Gemisch die (Meth)acrylsäure destillativ abtrennt, gefunden, das dadurch gekennzeichnet ist, daß man der Roh-(Meth)acrylsäure vor der destillativen Behandlung zusätzlich zum zugesetzten Carbonsäurehydrazid und/oder dessen Salzen wenigstens eine organische Sulfonsäure und/oder deren Salze (insbesondere die Alkalimetallsalze) zusetzt. Von Vorteil ist es, wenn die organische Sulfonsäure so beschaffen ist, daß sie bei Zusatz zu Wasser bei 25°C die Oberflächenspannung desselben reduziert. In der Regel wird man pro Mol zugesetzter Carbonsäurehydrazidverbindung wenigstens 0,1 Mol, üblicherweise aber nicht mehr als 5 Mol wenigstens einer organischen Sulfonsäure zusetzen, da oberhalb dieser Zusatzmenge keine verbesserte Wirkung erzielt wird. Vorzugsweise werden pro Mol zugesetzter Carbonsäurehydrazidverbindung 0,5 bis 2 mol wenigstens einer organischen Sulfonsäure zugesetzt. Wahlweise kann die Zugabe der organischen Sulfonsäure vor, zeitgleich oder nach der Zugabe der Carbonsäurehydrazidverbindung erfolgen. Ferner kann der Zusatz der organischen Sulfonsäure auch nur auf den Böden der Destillationskolonne, am Kolonnenkopf oder über den Rücklauf zur Kolonne erfolgen. Der Zusatz der organischen Sulfonsäure ist auch dann erfolgreich, wenn bereits Belagsbildung erfolgt ist.

Geeignete organische Sulfonsäuren sind z.B. Alkylsulfonsäuren der allgemeinen Formel

R¹―SO₃H,

in der R¹ für C₁- bis C₂₀-Alkyl steht. Ferner eignen sich die Salze dieser Alkylsulfonsäuren, insbesondere die Alkalimetallsalze. Ein bevorzugter Vertreter ist die Methansulfonsäure und deren Salze.

Ferner eignen sich Arylsulfonsäuren und deren Salze. Geeignete Arylsulfonsäureverbindungen sind Toluolsulfonsäuren wie p-Toluolsulfonsäure, Benzolsulfonsäure, Phenolsulfonsäuren, Xylolsulfonsäuren, Dibutylnaphthalinsulfonsäuren und deren Salze, insbesondere deren Alkalimetallsalze. Ganz besonders eignen sich jedoch Alkylarylsulfonsäuren und deren Salze, unter denen wiederum jene bevorzugt sind, die nur einen Alkylsubstituenten aufweisen. Aryl steht vorzugsweise für ein Benzol- oder Naphtalin-Ringsystem.

Mit Vorteil weist der Alkylrest 5 bis 16 C-Atome auf.

Von spezieller Eignung sind die Alkylbenzolsulfonsäuren und deren Salze, d.h. Verbindungen der allgemeinen Formel 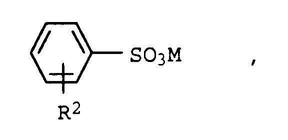 in der M für Wasserstoff oder ein anderes Kation, z.B. ein Alkalimetallion, und R² für C₅- bis C₁₆-Alkyl steht.

Vorzugsweise steht R² für C₈- bis C₁₂-Alkyl.

Unter den Alkylbenzolsulfonsäureverbindungen der oben angeführten allgemeinen Formel nehmen wiederum die Dodecylbenzolsulfonsäuren und deren Salze eine herausragende Position ein. Dies gilt insbesondere für Dodecylbenzolsulfonsäureverbindungen der allgemeinen Formel 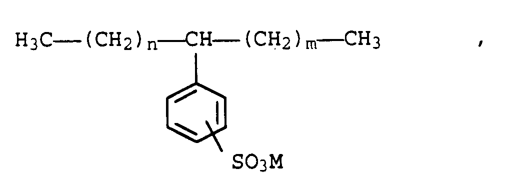 in der n, m ganze Zahlen bedeuten, deren Summe 9 ergeben muß. Ganz generell wird die Arylsulfonsäureverbindung vorzugsweise in ihrer vollsauren Form zugesetzt. D.h. die Arylsulfonsäure ist das bevorzugte Additiv. Dies gilt ebenso für die Alkylsulfonsäureverbindungen sowie für Aralkylsulfonsäureverbindungen, die sich ebenfalls als erfindungsgemäß zuzusetzende organische Sulfonsäureverbindung eignen.

Selbstverständlich läßt sich das erfindungsgemäße verfahren auch zur Abtrennung von Aldehyden aus (Meth)acrylsäure anwenden, bei der es sich nicht um Roh-(Meth)acrylsäure handelt, d.h. die auf anderem Weg als durch gasphasenkatalytische Oxidation von C₃-, C₄-Verbindungen erhalten worden und somit ursächlich aus anderen Gründen mit aldehydischen Verunreinigungen behaftet ist.

Vorzugsweise wird die destillative Aufarbeitung der erfindungsgemäß modifizierten Roh-(Meth)acrylsäure bei reduziertem Druck, zweckmäßigerweise ≤ 100 mbar, in der Regel 10 bis 100 mbar, vorgenommen. Entsprechend liegt die zugehörige Siedetemperatur üblicherweise im Bereich von 70 bis 105°C.

Von besonderer Bedeutung ist das erfindungsgemäße Verfahren im Fall von Roh-Methacrylsäure, deren gasphasenkatalytisch oxidative Herstellung ausgehend von Methacrolein erfolgt, insbesondere dann, wenn das Methacrolein durch gasphasenkatalytische Oxidation von tert.-Butanol, iso-Butan oder iso-Buten oder durch Umsetzung von Formaldehyd mit Propionaldehyd gemäß EP-B 92 097 oder EP-B 58 927 erzeugt wird, und dieses insbesondere dann, wenn die gasphasenkatalytische Oxidation des tert. Butanols, iso-Butans oder iso-Butens unter Verwendung einer katalytisch aktiven Masse der allgemeinen Formel I

Mo₁₂BiₐFe_{b}X¹_{c}X²_{d}X³ₑX⁴_{f}Oₙ (I),

in der die Variablen folgende Bedeutung haben:
- X¹: Nickel und/oder Kobalt,
- X²: Thallium, ein Alkalimetall und/oder ein Erdalkalimetall,
- X³: Phoshor, Arsen, Bor, Antimon, Zinn, Cer, Blei, Niob und/oder Wolfram,
- X⁴: Silicium, Aluminium, Titan und/oder Zirkonium,
- a: 0,5 bis 5,0
- b: 0,01 bis 3,0
- c: 3,0 bis 10,0
- d: 0,02 bis 2,0
- e: 0 bis 5,0
- f: 0 bis 10 und
- n: eine Zahl, die durch die Wertigkeit und Häufigkeit der von Sauerstoff verschiedenen Elemente in I bestimmt wird,
bei Temperaturen von 300 bis 400°C und, vom speziellen Temperaturverlauf abgesehen, ansonsten gemäß den Bedingungen der DE-A 40 23 239 erfolgt und das anfallende Methacrolein ohne Zwischenreinigung zur Weiteroxidation verwendet wird. Ferner bewährt sich das erfindungsgemäße Verfahren besonders dann, wenn die gasphasenkatalytische Oxidation des Methacroleins, vom speziellen Temperaturverlauf abgesehen, gemäß den Ausführungen der DE-A 41 32 263 bei Temperaturen von 200 bis 350°C bzw. gemäß der DE-A 41 32 684 bei Temperaturen von 250 bis 400°C erfolgt.

Selbstverständlich erfolgt das erfindungsgemäße Verfahren in Gegenwart von Polymerisationsinhibitoren wie Luft, Hydrochinon, Hydrochinonmonoethylether, Paranitrosophenol, Paramethoxyphenol und/oder Phenothiazin.

Üblicherweise werden sie, bezogen auf die Roh(Meth)acrylsäure, in Mengen von 50 bis 1000 ppm eingesetzt.

Besonders vorteilhaft hat sich das erfindungsgemäße Verfahren in Gegenwart von Phenothiazin und/oder aromatischen Hydroxyverbindungen wie Hydrochinon und Hydrochinonmonomethylether als Polymerisationsinhibitoren erwiesen.

Das erfindungsgemäße Verfahren ist dadurch ausgezeichnet, daß es einerseits eine besonders effektive destillative Abtrennung aldehydischer Verunreinigungen aus Roh- (Meth) acrylsäure bedingt und andererseits eine damit einhergehende Belagsbildung weitgehend unterdrückt. Das erfindungsgemäße Verfahren eignet sich daher z.B. nicht nur für eine absatzweise, sondern insbesondere für eine kontinuierliche Ausführungsweise, wie sie in der DE-A 42 01 697 beschrieben ist.

### Beispiele

### Beispiel 1

Eine Roh-Acrylsäure, die eine Gesamtmenge von 420 ppm (bezogen auf die Roh-Acrylsäure) an Furfural-2,Furfural-3 und Benzaldehyd enthielt, wurde bei 25°C mit 1100 ppm Aminoguanidinhydrogencarbonat versetzt und anschließend unter Aufrechterhaltung der 25°C sich selbst überlassen. Als Funktion der Zeit wurde dabei ihr Gehalt an vorgenannten Aldehyden gaschromatographisch ermittelt. Die erhaltenen Ergebnisse lauten:

| Zeit (min) | Aldehydgesamtmenge (ppm, bezogen auf Roh-Acrylsäure) |
|---|---|
| 5 | 380 |
| 30 | 250 |
| 60 | 80 |

### Beispiel 2

Wie Beispiel 1, anstelle des Aminoguanidinhydrogencarbonats wurde jedoch eine zu dessen 1100 ppm äquimolare Menge an Adipinsäuredihydrazid zugesetzt. Als Ergebnis wurde erhalten:

| Zeit (min) | Aldehydgesamtmenge (ppm, bezogen auf Roh-Acrylsäure) |
|---|---|
| 5 | 300 |
| 30 | 100 |
| 60 | 10 |

### Beispiel 3

Wie Beispiel 1, anstelle des Aminoguanidinhydrogencarbonats wurde jedoch eine zu dessen 1100 ppm äquimolare Menge an Bernsteinsäuredihydrazid zugesetzt. Als Ergebnis wurde erhalten:

| Zeit (min) | Aldehydgesamtmenge (ppm, bezogen auf Roh-Acrylsäure) |
|---|---|
| 5 | 20 |
| 30 | 10 |
| 60 | 10 |

### Beispiel 4

In eine kontinuierlich zu betreibende Rektifikationskolonne aus Glas, deren Verdampfer ein Konvektionsumlaufverdampfer aus Glas war, der mittels einer metallischen, elektrisch beheizbaren Kerze beheizt wurde, wurden über den Verdampfer kontinuierlich 140 ml/h einer Roh-Acrylsäure zugeführt, die durch katalytische Gasphasenoxidation von Acrolein gemäß Beispiel B1 der DE-A 43 02 991 und anschließende Aufarbeitung der Reaktionsgase gemäß Beispiel B1 der DE-A 21 36 396 erhalten worden war. Die gleiche Menge an Rohsäure wurde dem Verdampfer kontinuierlich entnommen. Die verdampfte Rohsäure wurde oberhalb der Rektifikationskolonne kondensiert und als Rücklauf quantitativ wieder auf den Kolonnenkopf gegeben. Eine Stabilisierung der Kolonne erfolgte mittels Phenothiazin über den Kolonnenkopf. Der Roh-Acrylsäure waren vor der Zufuhr in den Verdampfer 1400 ppm ihres Gewichtes an Adipinsäuredihydrazid zugesetzt worden. Während der Rektifikation trat auf der Heizkerze Belagsbildung auf, deren Menge nach einer Betriebsdauer von 8 h ausgewogen wurde. Das Ergebnis zeigt die nachfolgende Tabelle. Sie zeigt ferner das Ergebnisse das erhalten wurde, wenn der Roh-Acrylsäure unmittelbar vor ihrem Zulauf in den Verdampfer pro Mol enthaltenem Adipinsäuredihydrazid 1,5 mol Dodecylbenzolsulfonsäure zugesetzt wurden (nach 40 h).

**Tabelle**

| | |
|---|---|
| kein Zusatz an Sulfonsäure | 2400 mg Belag nach 8 h |
| mit Dodecylbenzolsulfonsäure | < 10 mg Belag nach 40 h |

## Patentansprüche

1. Verfahren zur Reinigung einer mit Aldehyden verunreinigten (Meth)acrylsäure, bei dem man die (Meth)acrylsäure mit einem Hydrazinderivat versetzt und aus dem Gemisch die (Meth)acrylsäure destillativ abtrennt, dadurch gekennzeichnet, daß man als Hydrazinderivat ein Carbonsäurehydrazid und/oder dessen Salz verwendet.

2. Verfahren nach Anspruch 1, dadurch gekennzeichnet, daß man als Hydrazinderivat das Hydrazid einer 1 bis 10 C-Atome aufweisenden gesättigten aliphatischen Mono- und/oder Dicarbonsäure verwendet.

3. Verfahren nach Anspruch 1 oder 2, dadurch gekennzeichnet, daß man als Carbonsäurehydrazid das Dihydrazid der Adipinsäure oder Bernsteinsäure verwendet.

4. Verfahren nach Anspruch 1 bis 3, dadurch gekennzeichnet, daß man pro Mol aldehydischer Verunreinigung 0,5 bis 5 Mol organische Carbonsäurehydrazidverbindung zusetzt.

5. Verfahren nach Anspruch 1 bis 4, dadurch gekennzeichnet, daß die mit Aldehyden verunreinigte (Meth)acrylsäure eine Roh-(Meth)acrylsäure ist, die nach dem Verfahren der katalytischen Gasphasenoxidation erzeugt worden ist.

6. Verfahren nach Anspruch 1 bis 5, dadurch gekennzeichnet, daß es kontinuierlich ausgeführt wird.

7. Verfahren nach Anspruch 1 bis 6, dadurch gekennzeichnet, daß man der mit Aldehyden verunreinigten (Meth)acrylsäure vor der destillativen Behandlung zusätzlich zum zugesetzten Carbonsäurehydrazid und/oder dessen Salzen wenigstens eine organische Sulfonsäure und/oder eines deren Salze zusetzt.

8. Verfahren nach Anspruch 1 bis 7, dadurch gekennzeichnet, daß man pro Mol zugesetzter Carbonsäurehydrazidverbindung bis zu 5 Mol wenigstens einer organischen Sulfonsäure und/oder eines deren Salze zusetzt.

9. Verfahren nach Anspruch 1 bis 8, dadurch gekennzeichnet, daß man pro Mol aldehydischer Verunreinigungen 1 bis 3 Mol an Carbonsäurehydrazid und/oder dessen Salzen, sowie pro Mol zugesetzter Carbonsäurehydrazidverbindung 0,5 bis 2 Mol wenigstens einer organischen Sulfonsäure und/oder eines deren Salze zusetzt.

10. Verfahren nach Anspruch 1 bis 9, dadurch gekennzeichnet, daß die zugesetzte Sulfonsäureverbindung Dodecylbenzolsulfonsäure umfaßt.

## Claims

1. A process for purifying an aldehyde-contaminated (meth)acrylic acid by admixing the (meth)acrylic acid with a hydrazine derivative and distilling the (meth)acrylic acid from the mixture, wherein the hydrazine derivative used is a carboxylic acid hydrazide and/or the salt thereof.

2. A process as claimed in claim 1, wherein the hydrazine derivative used is the hydrazide of a saturated aliphatic monocarboxylic and/or dicarboxylic acid having from 1 to 10 carbon atoms.

3. A process as claimed in claim 1 or 2, wherein the carboxylic acid hydrazide used is the dihydrazide of adipic acid or succinic acid.

4. A process as claimed in any of claims 1 to 3, wherein from 0.5 to 5 mol of organic carboxylic acid hydrazide compound is used per mole of aldehydic contamination.

5. A process as claimed in any of claims 1 to 4, wherein the aldehyde-contaminated (meth)acrylic acid is a crude (meth)acrylic acid which has been produced by the catalytic gas-phase oxidation process.

6. A process as claimed in any of claims 1 to 5, which is carried out continuously.

7. A process as claimed in any of claims 1 to 6, wherein the aldehyde-contaminated (meth)acrylic acid has added to it, prior to the distillative treatment, at least one organic sulfonic acid and/or a salt thereof in addition to the carboxylic acid hydrazide and/or salts thereof added.

8. A process as claimed in any of claims 1 to 7, wherein up to 5 mol of at least one organic sulfonic acid and/or a salt thereof is added per mole of carboxylic acid hydrazide compound added.

9. A process as claimed in any of claims 1 to 8, wherein from 1 to 3 mol of carboxylic acid hydrazide and/or salts thereof are added per mole of aldehydic contaminants, and from 0.5 to 2 mol of at least one organic sulfonic acid and/or a salt thereof are added per mole of carboxylic acid hydrazide compound added.

10. A process as claimed in any of claims 1 to 9, wherein the sulfonic acid compound added comprises dodecylbenzenesulfonic acid.

## Revendications

1. Procédé de purification d'acide (méth)acrylique pollué par des aldéhydes, au cours duquel on ajoute un dérivé de l'hydrazine à l'acide (méth)acrylique et on sépare l'acide (méth)acrylique par distillation du mélange, caractérisé en ce que l'on utilise comme dérivé de l'hydrazine un hydrazide d'acide carboxylique et/ou ses sels.

2. Procédé selon la revendication 1, caractérisé en ce que l'on utilise, comme dérivé de l'hydrazine, l'hydrazide d'un acide mono- et/ou dicarboxylique aliphatique saturé à 1-10 atomes de carbone.

3. Procédé selon la revendication 1 ou 2, caractérisé en ce que l'on utilise, comme hydrazide d'acide carboxylique, le dihydrazide d'acide adipique ou d'acide succinique.

4. Procédé selon l'une quelconque des revendications 1 à 3, caractérisé en ce que l'on introduit 0,5-5 moles de composé organique hydrazide d'acide carboxylique par mole d'impureté aldéhydique.

5. Procédé selon l'une quelconque des revendications 1 à 4, caractérisé en ce que l'acide (méth)acrylique pollué par des aldéhydes est un acide (méth)acrylique brut, obtenu par le procédé d'oxydation catalytique en phase gazeuse.

6. Procédé selon l'une quelconque des revendications 1 à 5, caractérisé en ce qu'il est mené de façon continue.

7. Procédé selon l'une quelconque des revendications 1 à 6, caractérisé en ce que l'on ajoute à l'acide (méth)acrylique pollué par des aldéhydes, avant son traitement par distillation, au moins un acide sulfonique organique et/ou un de ses sels, en plus de l'hydrazide d'acide carboxylique et/ou de ses sels.

8. Procédé selon l'une quelconque des revendications 1 à 7, caractérisé en ce que l'on ajoute jusqu'à 5 moles d'au moins un acide sulfonique organique et/ou d'un de ses sels, par mole de composé hydrazide d'acide carboxylique ajoutée.

9. Procédé selon l'une quelconque des revendications 1 à 8, caractérisé en ce que l'on ajoute 1-3 moles d'hydrazide d'acide carboxylique et/ou de ses sels par mole d'impuretés aldéhydiques, ainsi que 0,5-2 moles d'au moins un acide sulfonique organique et/ou d'un de ses sels par mole de composé hydrazide d'acide carboxylique ajoutée.

10. Procédé selon l'une quelconque des revendications 1 à 9, caractérisé en ce que le composé acide sulfonique ajouté comprend l'acide dodécylbenzènesulfonique.
